# FASCICULE DE BREVET EUROPEEN

(11) **EP 2 590 699 B1**
(45) Date de publication et mention de la délivrance du brevet: **21.05.2014**
(21) Numéro de dépôt: 11743086.8
(22) Date de dépôt: 05.07.2011
(51) Int. Cl.: A61M 15/00

(54) **DISPOSITIF DE DISTRIBUTION DE PRODUIT FLUIDE**
SPENDEVORRICHTUNG FÜR EIN FLUIDES PRODUKT
DEVICE FOR DISPENSING A FLUID PRODUCT

(30) Priorité: 07.07.2010 FR 1055527
(43) Date de publication de la demande: 15.05.2013
(73) Titulaire: Aptar France SAS, 27110 Le Neubourg (FR)
(72) Inventeur: KIRNIAK, Maxime, F-76000 Rouen (FR)
(74) Mandataire: CAPRI
(86) Numéro de dépôt international: PCT/FR2011/051576
(87) Numéro de publication internationale: WO 2012/004509

(56) Documents cités:
- WO-A1-2009/077697
- WO-A2-2008/012456

## Description

La présente invention concerne un dispositif de distribution de produit fluide, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Un autre problème qui se pose avec les inhalateurs pourvus de bande de blisters est lié au déplacement de la bande, et au stockage de la partie utilisée de la bande. Ainsi, selon la longueur de la bande et/ou l'épaisseur des blisters, un espace important peut s'avérer nécessaire et tout blocage de la bande de blisters peut empêcher le bon fonctionnement de l'inhalateur. Par ailleurs, lorsque le dispositif d'avancée de la bande tire en même temps sur l'extrémité avant de la bande pour éviter un mauvais enroulement, il peut se poser un problème au fur et à mesure des actionnements en raison notamment du diamètre de la bande usée enroulée qui augmente progressivement. Les documents WO 2008/012456 et WO 2009/077697 décrivent des dispositifs de l'art antérieur.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel dispositif qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a donc pour objet un dispositif de distribution de produit fluide, comportant :
- un corps pourvu d'un orifice de distribution,
- au moins un élément de capot mobile entre une position de fermeture et une position d'ouverture,
- une pluralité de blisters individuels contenant chacun une dose de produit fluide, telle que de la poudre pharmaceutique, lesdits blisters étant formés sur une bande de blisters allongée,
- des moyens d'ouverture de blister pour ouvrir un blister plein respectif à chaque actionnement desdits moyens d'ouverture,
- des premiers moyens de déplacement de ladite bande de blisters pour amener un blister plein en face desdits moyens d'ouverture avant chaque inhalation, lesdits premiers moyens de déplacement comportant une roue d'indexage pourvue d'au moins une cavité recevant les blisters,
- des seconds moyens de déplacement de ladite bande de blisters, pivotant entre une position de non-distribution et une position de distribution, dans laquelle un blister coopère avec lesdits moyens d'ouverture, ladite roue d'indexage étant montée rotative sur lesdits seconds moyens de déplacement, de sorte qu'en position de distribution, ladite roue d'indexage est déplacée contre lesdits moyens d'ouverture pour ouvrir le blister disposé dans ladite cavité,
- un organe d'armement pour solliciter lesdits seconds moyens de déplacement vers ladite position de distribution, ledit organe d'armement étant chargé en comprimant un ressort par l'ouverture dudit au moins un élément de capot,
- des moyens de blocage pour maintenir lesdits seconds moyens de déplacement en position de non-distribution,
- des moyens de déclenchement, pour libérer lesdits moyens de blocage et permettre le déplacement desdits seconds moyens de déplacement, et donc de ladite roue d'indexage, vers ladite position de distribution, lesdits moyens de déclenchement étant actionnés par l'inhalation de l'utilisateur,
- un chariot coulissant déplaçable entre une position de non-indexage et une position d'indexage, ladite position de non-indexage correspondant à la position ouverte dudit au moins un élément de capot et la position d'indexage correspondant à ladite position fermée dudit au moins un élément de capot, ledit chariot comportant un organe actionneur coopérant avec ladite roue d'indexage pour la faire tourner lorsque ledit chariot se déplace de sa position d'indexage vers sa position de non-indexage,
- des moyens de solidarisation, qui lorsqu'ils sont activés, solidarisent ledit chariot avec ledit au moins un élément de capot pour déplacer ledit chariot vers sa position d'indexage lorsque ledit au moins un élément de capot est ramené de sa position d'ouverture vers sa position de fermeture, et pour déplacer ledit chariot vers sa position de non-indexage lorsque ledit au moins un élément de capot est ramené de sa position de fermeture vers sa position d'ouverture,
- lesdits moyens de solidarisation étant activés lorsque lesdits seconds moyens de déplacement sont déplacés vers ladite position de distribution lors de l'inhalation de l'utilisateur, et étant désactivés en l'absence d'inhalation, de telle sorte qu'une ouverture suivie d'une fermeture dudit au moins un capot mobile, sans inhalation avant la fermeture, ne provoque pas de rotation de ladite roue d'indexage.

Avantageusement, lesdits moyens de solidarisation comportent un doigt de solidarisation déplaçable ensemble avec ledit organe d'armement.

Avantageusement, ladite roue d'indexage comporte une denture coopérant avec l'organe actionneur dudit chariot lorsque ledit chariot se déplace de sa position d'indexage vers sa position de non indexage.

Avantageusement, le dispositif comporte deux éléments de capots engrenés l'un avec l'autre.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- les figures 1 à 4 sont des vues en section transversale d'un dispositif de distribution selon un mode de réalisation avantageux de l'invention, lors d'un cycle d'ouverture puis de fermeture du dispositif, sans que l'utilisateur n'ait inhalé en position d'ouverture,
- les figures 5 à 10 représentent des vues similaires à celles de la figure 1, lors d'un cycle d'ouverture puis de fermeture du dispositif, avec l'utilisateur qui inhale en position d'ouverture,
- les figures 11 à 13 sont des vues similaires à celles des figures 5 et 6, montrant un cycle d'ouverture du dispositif après une inhalation lors du cycle précédent, et
- les figures 14 à 16 représentent des vues schématiques partielles des figures 11 à 13.

Sur les figures est représenté un exemple avantageux d'un inhalateur de poudre sèche. Cet inhalateur comporte un corps 10 sur lequel peuvent être montées coulissantes deux parties 11, 12 formant capot (seulement partiellement représentées dans des buts de clarté) adaptées à être ouvertes pour ouvrir et charger le dispositif. Le corps 10 peut être de forme environ arrondie comme représenté sur les figures, mais il pourrait avoir tout autre forme appropriée. Le corps 10 comporte un embout buccal ou d'inhalation 5 définissant un orifice de distribution à travers lequel l'utilisateur va inhaler lors de l'actionnement du dispositif. Cet orifice est typiquement disposé environ au centre de la partie supérieure (dans la position représentée sur les dessins). Les capots 11, 12 peuvent s'ouvrir par pivotement autour d'un axe de rotation commun, ou autour de deux axes parallèles en étant engrené l'un avec l'autre. Tout autre moyen d'ouverture du dispositif est envisageable. En variante, le dispositif pourrait ne comporter qu'un seul capot au lieu de deux.

A l'intérieur du corps 10, il est prévu une bande de réservoirs individuels 21, également appelés blisters, réalisée sous forme d'une bande allongée 20 sur laquelle les blisters 21 sont disposés les uns derrière les autres, de manière connue. Cette bande de blisters est avantageusement constituée d'une couche ou paroi de base formant les cavités recevant les doses de poudre, et d'une couche ou paroi de fermeture qui obture de manière étanche chacun desdits blisters. Avant la première utilisation, la bande de blisters peut être enroulée à l'intérieur du corps 10, de préférence dans une partie de stockage, et des premiers moyens de déplacement de bande 40 rotatifs sont prévus pour progressivement dérouler et faire avancer cette bande de blisters. Des seconds moyens de déplacement, pivotants sur le corps 10, sont prévus pour amener un blister respectif dans une position de distribution à chaque actionnement du dispositif. Ces seconds moyens de déplacement sont pivotants entre une position de non-distribution et une position de distribution, dans laquelle un blister coopère avec lesdits moyens d'ouverture. La partie de bande comportant les blisters vides est avantageusement adaptée à s'enrouler dans un autre endroit dudit corps 10, de préférence une partie de réception, comme cela sera décrit plus en détail ci-après.

L'inhalateur comporte des moyens d'ouverture de blister (non représentés dans des buts de clarté) comportant de préférence une aiguille de perçage et/ou de coupage de la couche de fermeture des blisters. Par exemple, les moyens d'ouverture comportent une aiguille fixe par rapport au corps 10, et contre laquelle un blister respectif est déplacé à chaque actionnement par les seconds moyens de déplacement. Le blister est alors percé par ladite aiguille, qui pénètre dans ledit blister pour expulser la poudre au moyen du flux d'inhalation de l'utilisateur.

Les premiers moyens de déplacement 40 sont adaptés à faire avancer la bande de blisters après chaque inhalation de l'utilisateur. Les seconds moyens de déplacement sont adaptés à déplacer le blister à vider contre lesdits moyens d'ouverture lors de l'actionnement, avant chaque inhalation. Ces seconds moyens de déplacement sont sollicités par un ressort qui est préchargé lors de l'ouverture du dispositif. Les premiers moyens de déplacement sont formés par une roue d'indexage 40 qui reçoit et guide la bande de blisters. Une rotation de cette roue d'indexage 40 fait avancer la bande de blisters. Avant chaque inhalation, un blister plein est toujours en position face aux moyens d'ouverture. Les seconds moyens de déplacement peuvent comporter un organe pivotant autour d'un axe de rotation, ladite roue d'indexage 40 étant montée rotative sur ledit organe pivotant.

Un cycle d'actionnement du dispositif peut être le suivant. Lors de l'ouverture du dispositif, les deux parties latérales 11, 12 formant capot sont écartées l'une de l'autre en pivotant sur le corps pour ouvrir le dispositif, et ainsi charger le dispositif. Dans cette position la roue d'indexage 40 ne peut pas se déplacer vers l'aiguille car les seconds moyens de déplacement sont retenus par des moyens de blocage 30 appropriés. Lors de l'inhalation par l'utilisateur à travers l'embout buccal, ces moyens de blocage sont débloqués, ce qui provoque alors le déplacement de ladite roue d'indexage 40 en direction de l'aiguille, et donc l'ouverture d'un blister.

De préférence, le blister se déplace vers sa position d'ouverture pour être ouvert par l'aiguille qui est fixe par rapport au corps 10. Toutefois, il est envisageable que cette aiguille puisse également être mobile pendant la phase d'ouverture du blister. Par exemple, l'aiguille pourrait se déplacer en direction du blister pendant que le blister se déplace en direction de l'aiguille. Dans une autre variante, on pourrait également envisager que le blister et l'aiguille se déplacent dans la même direction lors de l'actionnement, le blister se déplaçant plus rapidement dans cette direction de sorte qu'il vient en contact avec l'aiguille pour être ouvert.

L'actionnement des moyens d'ouverture est réalisé par l'inhalation de l'utilisateur. Pour réaliser ce déclenchement par inhalation des moyens d'ouverture, on peut prévoir un système de déclenchement par l'inhalation qui comporte avantageusement une unité 60 déplaçable et/ou déformable sous l'effet de l'inhalation, cette unité étant adaptée à libérer les moyens de blocage. Cette unité comprend avantageusement une chambre d'air ou diaphragme déformable 61. L'inhalation de l'utilisateur provoque la déformation de ladite chambre d'air déformable, permettant ainsi de libérer lesdits moyens de blocage et donc de permettre le déplacement des seconds moyens de déplacement, et donc d'un blister respectif, vers sa position d'ouverture. Le blister n'est donc ouvert qu'au moment de l'inhalation, de sorte qu'il est simultanément vidé. Il n'y a donc aucun risque de perte de dose entre l'ouverture du blister et son vidage.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage et donc le déplacement du blister vers les moyens d'ouverture.

L'inhalateur comporte en outre une chambre de distribution ou dispersion 70 qui est destinée à recevoir la dose de poudre après ouverture d'un blister respectif. Avantageusement, cette chambre de distribution est pourvue d'au moins une bille, de préférence plusieurs, qui se déplace à l'intérieur de ladite chambre pendant l'inhalation, pour améliorer la distribution du mélange air et poudre après ouverture d'un blister, afin d'augmenter l'efficacité du dispositif.

Il peut être avantageux que les moyens d'ouverture, en particulier l'aiguille, soient formés directement sur ladite chambre de distribution, par exemple à l'extrémité d'un canal menant à ladite chambre.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les blisters sont formés sur une bande allongée souple, qui, au début, est principalement stockée sous forme de bobine dans un logement de stockage à l'intérieur du corps 10 du dispositif. Avantageusement, cette bande de blisters enroulée est maintenue par des parois internes dudit logement de stockage sans que son extrémité arrière (dans le sens d'avancement de la bande de blisters) ne soit fixée par rapport audit corps 10, ce qui permet un assemblage plus aisé de cette bobine de bande de blisters à l'intérieur du dispositif. La bande de blisters est déplacée au moyen de la roue d'indexage 40 qui présente au moins un, de préférence plusieurs évidements, dont la forme correspond à celle des blisters. Ainsi, lorsque cette roue d'indexage 40 tourne, elle fait avancer la bande de blisters. Bien entendu de manière additionnelle, on pourrait utiliser d'autres moyens d'avancée de bande de blisters, par exemple en prévoyant un profil sur les bords longitudinaux latéraux de la bande de blisters, ledit profil étant adapté à coopérer avec des moyens d'entraînement appropriés. De même, des trous ménagés le long des bords latéraux de la bande de blisters pourraient également être utilisés pour faire avancer la bande de blisters au moyen de roues dentées coopérant avec lesdits trous.

Après ouverture d'un ou plusieurs blister(s), la partie de bande de blisters avec les blisters vidés doit pouvoir être stockée de manière aisée et non encombrante dans le dispositif, en évitant tout risque de blocage. Avantageusement, cette bande de blisters usée s'enroule automatiquement sur elle-même pour à nouveau former une bobine.

Selon encore un autre aspect de l'inhalateur, un dispositif de comptage ou d'indication de doses (non représenté) est également prévu. Ce dispositif peut comporter des chiffres ou symboles inscrits directement sur la bande de blister et visibles à travers une fenêtre appropriée dans le corps 10 du dispositif. En variante, on pourrait envisager d'utiliser un ou plusieurs disque(s) ou anneau(x) rotatif(s) comportant des numéros ou symboles.

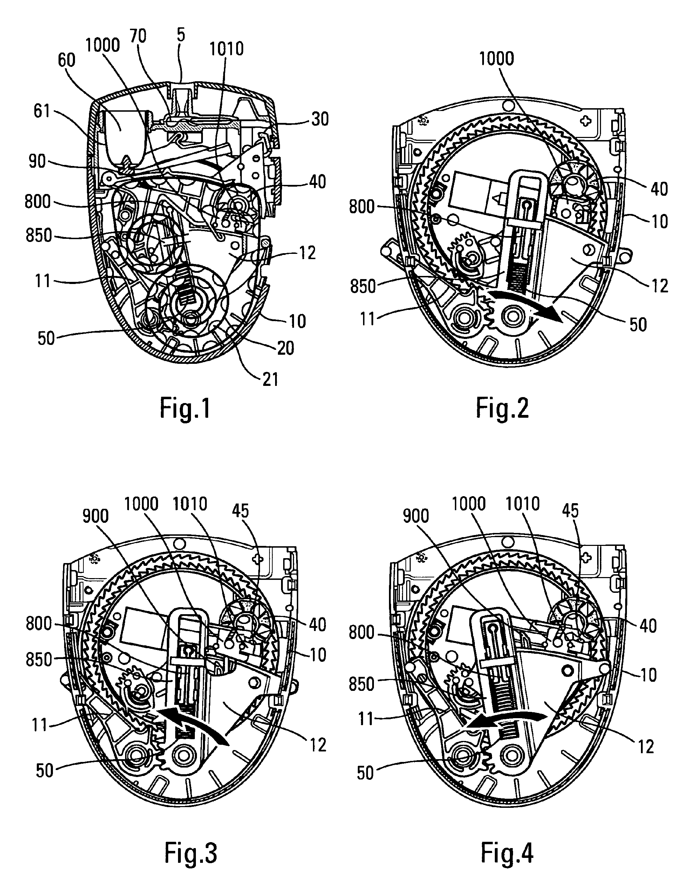

Les figures 1 à 4 représentent un cycle d'ouverture et de fermeture du dispositif, sans que l'utilisateur n'inhale en position d'ouverture. Les déplacements de certaines pièces sont indiqués schématiquement par des flèches sur ces figures.

L'élément de capot mobile 12 supporte un organe d'armement 800, avantageusement réalisé sous la forme d'une tige, qui peut coulisser dans un logement 850 solidaire dudit élément de capot 12. Cet organe d'armement 800 est donc pivotant par rapport audit corps 10 ensemble avec l'élément de capot 12. Cet organe d'armement 800 peut se déplacer contre un ressort 50, avantageusement un ressort à boudins, disposé dans ledit logement 850. L'organe d'armement 800 est donc connecté d'un côté audit ressort 50 et de l'autre côté, il coopère avec les seconds moyens de déplacement, en particulier avec un organe 90 pivotant sur le corps 10, et sur lequel est fixée de manière rotative la roue d'indexage 40. Lorsque l'élément de capot mobile 12 est ouvert, comme représenté sur les figures 1 (position fermée) et 2 (position ouverte), l'organe d'armement 800 est déplacé dans son logement 850 en comprimant le ressort 50. L'organe pivotant 90 des seconds moyens de déplacement est lui empêché de se déplacer par les moyens de blocage 30, qui ne seront libérés qu'au moment de l'inhalation. Ainsi, en l'absence d'inhalation dans la position ouverte de la figure 2, la fermeture des éléments de capot provoque simplement le retour à la position de repos pour l'organe d'armement 800, et la décompression du ressort 50. Ceci est représenté sur les figures 3 et 4. Avantageusement, la tige 800 comporte, dans sa partie en contact avec la l'organe pivotant, une partie arrondie, telle qu'une extrémité en forme de boule, pour favoriser le glissement de la tige 800 sur la pièce avec laquelle elle coopère.

Ainsi, lorsque l'utilisateur ouvre l'inhalateur il charge le système (figures 1 & 2). S'il n'inhale pas et qu'il referme l'inhalateur, celui-ci reprend simplement sa position de départ sans déplacer la bande de blisters ni les moyens de blocage (figures 3 & 4). Il n'y a donc aucun risque qu'un blister (et donc une dose de produit actif) soit perdu par un actionnement malencontreux ou incomplet dans lequel l'utilisateur n'exercerait pas d'inhalation entre l'ouverture et la fermeture. L'ouverture du blister, son vidage, la distribution de la poudre dans les poumons de l'utilisateur, le déplacement de la bande de blisters pour amener un nouveau blister plein en face des moyens d'ouverture ainsi que le comptage de la dose ne sont donc possibles que si l'utilisateur inhale.

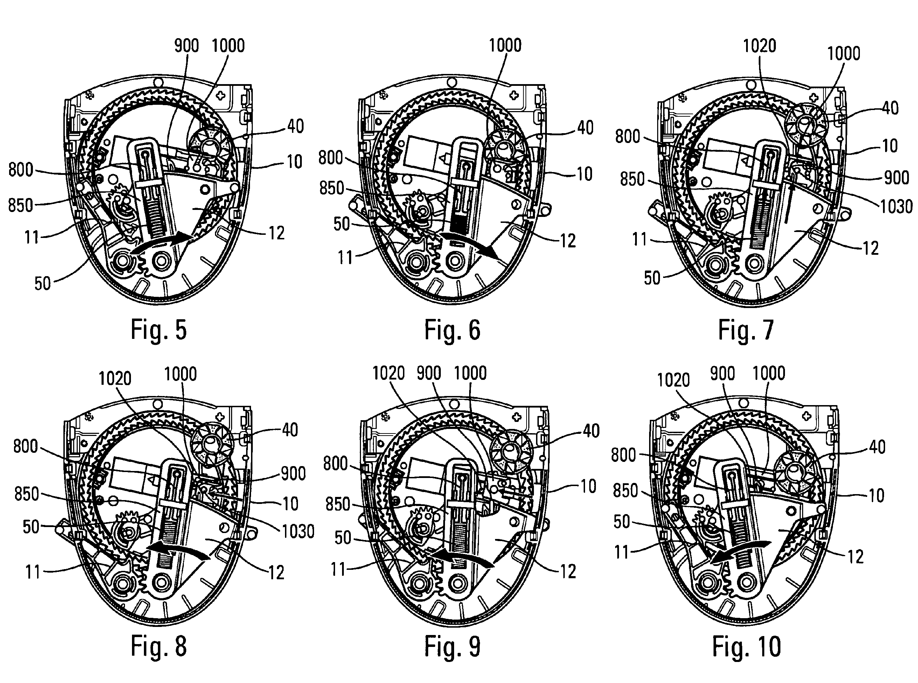

Les moyens de blocage 30, qui bloquent les seconds moyens de déplacement et notamment l'organe pivotant qui coopère avec l'organe d'armement 800, sont connectés au diaphragme déformable 61 sensible à l'inhalation de l'utilisateur, de sorte que lors de l'inhalation de l'utilisateur, ledit diaphragme se déforme, libérant ainsi lesdits moyens de blocage. Ceci permet le déplacement desdits seconds moyens de déplacement vers leur position de distribution sous l'effet de la force exercée par le ressort comprimé 50. Ce déplacement provoque l'ouverture d'un blister plein et la distribution d'une dose. Les figures 5 à 7 illustrent l'ouverture du dispositif suivie d'une inhalation.

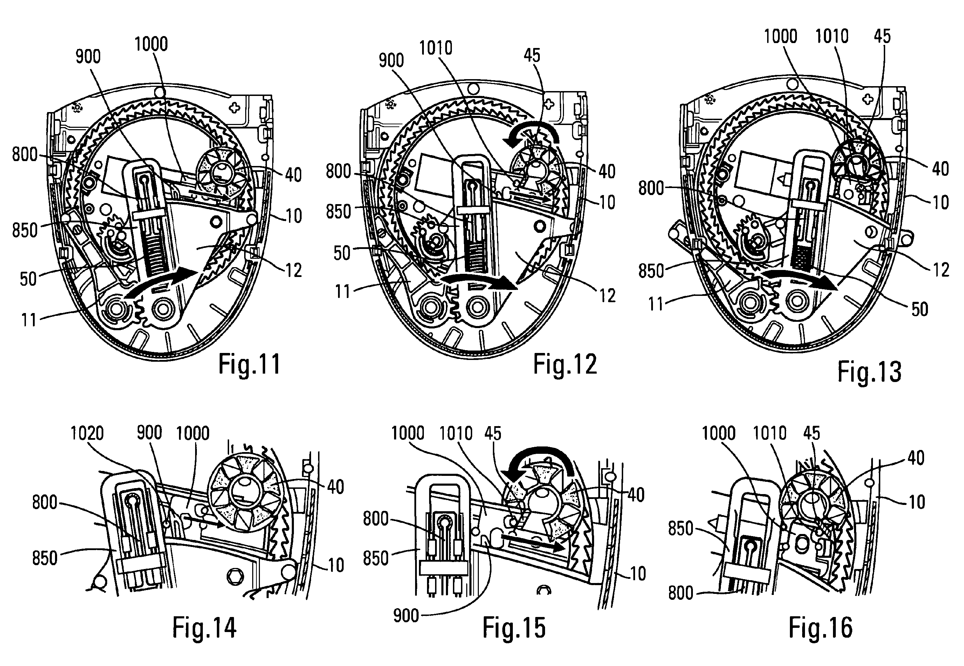

Pour réaliser la rotation de la roue d'indexage 40, et donc pour faire avancer la bande de blisters et amener un prochain blister face à l'aiguille des moyens d'ouverture, le dispositif comprend un chariot coulissant 1000. Ce chariot peut coulisser environ transversalement ou horizontalement dans la position du dispositif représenté sur les figures. Un doigt de solidarisation 900 est associé à l'organe d'armement 800, et se déplace avec lui. Ainsi, lorsque l'organe d'armement 800 coulisse dans son logement 850 en comprimant le ressort 50 lors de l'ouverture du dispositif, ledit doigt de solidarisation 900 coulissera aussi dans le même sens. En position ouverte, ce doigt de solidarisation 900 ne coopère pas avec ledit chariot 1000 tant que l'utilisateur n'a pas inhalé. Ainsi, si l'utilisateur n'inhale pas après avoir ouvert les éléments de capot, et qu'il referme ceux-ci, le doigt de solidarisation ne se solidarisera pas avec ledit chariot, et ledit chariot ne sera pas déplacé par l'ouverture puis la fermeture desdits éléments de capot. Ce n'est que lorsque l'utilisateur inhale dans la position ouverte (figures 6 & 7) que ledit doigt de solidarisation 900 se déplacera ensemble avec ledit organe d'armement 800, sous l'effet du ressort 50, pour s'engrener ou autrement coopérer avec ledit chariot 1000. Dans le mode de réalisation représenté, le doigt de solidarisation 900 s'insère entre deux projections latérales 1020, 1030 dudit chariot 1000, comme visible sur la figure 7 notamment. Ainsi, après inhalation, le doigt 900 est en position de solidarisation avec ledit chariot 1000. Lorsque l'utilisateur referme à nouveau les éléments de capot après avoir inhalé, comme illustré sur les figures 8 à 10, ledit élément de capot 12 qui supporte l'organe d'armement 800 et le doigt de solidarisation 900 pivotera vers la gauche sur les figures, comme schématisé par les grosses flèches courbes. Le doigt de solidarisation 900, en suivant ce mouvement de pivotement, entrainera le chariot 1000 à coulisser vers la gauche sur les figures, comme illustré par les petites flèches droites sur les figures 8 et 9. Simultanément, lors de cette fermeture, lesdits seconds moyens de déplacement reviennent aussi vers leur position non actionnée, ramenant la roue d'indexage 40 en éloignement de l'aiguille des moyens d'ouverture. Alors qu'en position actionnée, ladite roue d'indexage 40 ne coopère pas avec ledit chariot 1000, en position de repos ou non actionnée, une denture 45 de ladite roue d'indexage se trouve sur la trajectoire d'une partie actionneur 1010 dudit chariot 1000. Ainsi, lorsque l'utilisateur ouvrira à nouveau les éléments de capot après un précédente cycle avec inhalation, c'est-à-dire à partir de la position représentée sur les figures 10 et 11, ledit doigt de solidarisation 900 ramènera le chariot 1000 vers la droite sur les figures, vers sa position de départ, ce qui est symbolisé par les petites flèches rectilignes sur les figures 11 et 12. Lors de ce coulissement vers la droite, ledit chariot 1000 coopère avec la denture 45 de la roue d'indexage, provoquant ainsi la rotation de celle-ci pour ainsi faire avancer la bande de blisters et amener le prochain blister face à l'aiguille pour la prochaine inhalation. Les figures 14 à 16 montrent de manière plus détaillée la coopération entre l'organe actionneur 1010 du chariot et la denture 45 de la roue d'indexage. Simultanément, lors de cette ouverture, l'organe d'armement 800 est à nouveau armé, et donc le doigt de solidarisation 900 se désolidarise à nouveau dudit chariot 1000 en fin d'ouverture. Ainsi, si l'utilisateur referme sans inhaler, le chariot 1000 ne bougera pas, et il n'y aura pas de rotation supplémentaire de la roue d'indexage 40 lors de la prochaine ouverture. Ce n'est qu'après la prochaine inhalation qu'à nouveau le doigt de solidarisation 900 se solidarisera avec le chariot 1000, permettant un nouveau cycle d'indexage.

Afin d'éviter de compter des doses qui ne sont pas distribuées, par exemple en cas d'erreur de manipulation, ou d'une manipulation incomplète du dispositif, il est souhaitable que le compteur ou indicateur ne soit actionné qu'une fois que l'utilisateur a inhalé, puisque c'est cette inhalation qui conditionne l'ouverture et la distribution de la dose de chaque blister. Avantageusement, l'actionnement du compteur se fait donc après inhalation, lorsque l'utilisateur referme le dispositif.

Dans tous les modes de réalisation décrits ci-dessus, la bande de blisters est formée par une bande présentant deux extrémités. En variante, on pourrait utiliser une bande continue. D'autres modifications sont également possibles sans sortir du cadre de la présente invention.

L'inhalateur de poudre sèche tel que décrit précédemment procure notamment les fonctions suivantes:
■ une pluralité de doses individuelles de poudre stockées dans des blisters individuels étanches, par exemple 30 ou 60 doses stockées sur une bande enroulée en bobine ;
■ la poudre libérée au moyen d'un perçage actionné par l'inhalation de l'utilisateur, ce perçage du blister étant réalisé au moyen d'un système de détection d'inhalation couplé à un système de libération préchargé ;
■ des moyens d'entraînement de forme appropriée en prises avec les blisters pour réaliser le déplacement de la bande de blisters après chaque inhalation, et amener un nouveau blister plein dans une position dans laquelle il est destiné à être ouvert par les moyens d'ouverture appropriés ;
■ des moyens pour éviter une perte de dose en cas d'ouverture de l'inhalateur mais en l'absence d'inhalation ;
■ un indicateur de doses adapté à compter les doses seulement en cas d'inhalation.

D'autres fonctions sont également fournies par l'inhalateur tel que décrit précédemment. Il est à noter que les différentes fonctions, même si elles ont été représentées comme prévues simultanément sur l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les blisters individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Dispositif de distribution de produit fluide, comportant:
- un corps (10) pourvu d'un orifice de distribution,
- au moins un élément de capot (11, 12) mobile entre une position de fermeture et une position d'ouverture,
- une pluralité de blisters individuels (21) contenant chacun une dose de produit fluide, telle que de la poudre pharmaceutique, lesdits blisters étant formés sur une bande de blisters allongée (20),
- des moyens d'ouverture de blister pour ouvrir un blister plein respectif à chaque actionnement desdits moyens d'ouverture,
- des premiers moyens de déplacement de ladite bande de blisters pour amener un blister plein en face desdits moyens d'ouverture avant chaque inhalation, lesdits premiers moyens de déplacement comportant une roue d'indexage (40) pourvue d'au moins une cavité recevant les blisters,
- des seconds moyens de déplacement (90) de ladite bande de blisters, pivotant entre une position de non-distribution et une position de distribution, dans laquelle un blister coopère avec lesdits moyens d'ouverture, ladite roue d'indexage (40) étant montée rotative sur lesdits seconds moyens de déplacement, de sorte qu'en position de distribution, ladite roue d'indexage (40) est déplacée contre lesdits moyens d'ouverture pour ouvrir le blister disposé dans ladite cavité,
- un organe d'armement (800) pour solliciter lesdits seconds moyens de déplacement vers ladite position de distribution, ledit organe d'armement étant chargé en comprimant un ressort (50) par l'ouverture dudit au moins un élément de capot,
- des moyens de blocage (30) pour maintenir lesdits seconds moyens de déplacement en position de non-distribution,
- des moyens de déclenchement (60), pour libérer lesdits moyens de blocage et permettre le déplacement desdits seconds moyens de déplacement, et donc de ladite roue d'indexage, vers ladite position de distribution, lesdits moyens de déclenchement étant actionnés par l'inhalation de l'utilisateur,
ledit dispositif étant **caractérisé en ce qu'**il comporte :
- un chariot coulissant (1000) déplaçable entre une position de non-indexage et une position d'indexage, ladite position de non-indexage correspondant à la position ouverte dudit au moins un élément de capot et la position d'indexage correspondant à ladite position fermée dudit au moins un élément de capot, ledit chariot comportant un organe actionneur (1010) coopérant avec ladite roue d'indexage (40) pour la faire tourner lorsque ledit chariot se déplace de sa position d'indexage vers sa position de non-indexage,
- des moyens de solidarisation (900), qui lorsqu'ils sont activés, solidarisent ledit chariot (1000) avec ledit au moins un élément de capot (12) pour déplacer ledit chariot vers sa position d'indexage lorsque ledit au moins un élément de capot est ramené de sa position d'ouverture vers sa position de fermeture, et pour déplacer ledit chariot vers sa position de non-indexage lorsque ledit au moins un élément de capot est ramené de sa position de fermeture vers sa position d'ouverture,
- lesdits moyens de solidarisation (900) étant activés lorsque lesdits seconds moyens de déplacement sont déplacés vers ladite position de distribution lors de l'inhalation de l'utilisateur, et étant désactivés en l'absence d'inhalation, de telle sorte qu'une ouverture suivie d'une fermeture dudit au moins un capot mobile, sans inhalation avant la fermeture, ne provoque pas de rotation de ladite roue d'indexage.

2. Dispositif selon la revendication 1, dans lequel lesdits moyens de solidarisation comportent un doigt de solidarisation (900) déplaçable ensemble avec ledit organe d'armement (800).

3. Dispositif selon la revendication 1 ou 2, dans lequel ladite roue d'indexage (40) comporte une denture (45) coopérant avec l'organe actionneur (1010) dudit chariot (1000) lorsque ledit chariot se déplace de sa position d'indexage vers sa position de non indexage.

4. Dispositif selon l'une quelconque des revendications précédentes, comportant deux éléments de capots (11, 12) engrenés l'un avec l'autre.

## Patentansprüche

1. Ausgabevorrichtung für ein fluides Produkt, aufweisend:
- einen mit einer Ausgabeöffnung versehenen Körper (10),
- mindestens ein Abdeckelement (11, 12), das zwischen einer Schließposition und einer Öffnungsposition beweglich ist,
- eine Vielzahl von einzelnen Blisterpackungen (21), die jeweils eine Dosis eines fluiden Produktes wie ein pharmazeutisches Pulver enthalten, wobei die Blisterpackungen auf einem langgestreckten Blisterstreifen (20) ausgebildet sind,
- Blisteröffnungsmittel, um bei jeder Betätigung der Öffnungsmittel eine volle Blisterpackung zu öffnen,
- erste Verschiebemittel für den Blisterstreifen, um vor jeder Inhalation eine volle Blisterpackung in eine Position gegenüber den Öffnungsmitteln zu überführen, wobei die ersten Verschiebemittel ein Schaltrad (40) aufweisen, das mit mindestens einem, die Blisterpackungen aufnehmenden Hohlraum versehen ist,
- zweite Verschiebemittel (90) für den Blisterstreifen, die zwischen einer Position, in der keine Ausgabe stattfindet, und einer Ausgabeposition verschwenkbar sind, wobei eine Blisterpackung mit den Öffnungsmitteln zusammenwirkt, wobei das Schaltrad (40) drehbar auf den zweiten Verschiebemitteln montiert ist, so dass das Schaltrad (40) in der Ausgabeposition gegen die Öffnungsmittel verschoben wird, um die in dem Hohlraum angeordnete Blisterpackung zu öffnen,
- eine Spanneinrichtung (800), um die zweiten Verschiebemittel in die Ausgabeposition zu belasten, wobei die Spanneinrichtung belastet ist und dabei durch die Öffnung des mindestens einen Abdeckelementes eine Feder (50) zusammendrückt,
- Sperrmittel (30) zum Halten der zweiten Verschiebemittel in einer Position, in der keine Ausgabe stattfindet,
- Lösemittel (60) zum Freigeben der Sperrmittel und Ermöglichen einer Verschiebung der zweiten Verschiebemittel und somit des Schaltrades in die Ausgabeposition, wobei die Lösemittel durch Inhalation durch den Benutzer betätigt werden,
wobei die Vorrichtung **dadurch gekennzeichnet ist, dass** sie aufweist:
- einen verschiebbaren Wagen (1000), der zwischen einer Position ohne Schalten und einer Schaltposition verschiebbar ist, wobei die Position ohne Schalten der geöffneten Position des mindestens einen Abdeckelements entspricht und die Schaltposition der geschlossenen Position des mindestens einen Abdeckelements entspricht, wobei der Wagen eine Betätigungseinrichtung (1010) aufweist, die mit dem Schaltrad (40) zusammenwirkt, um es in Drehung zu versetzen, wenn sich der Wagen von seiner Schaltposition in seine Position ohne Schalten verschiebt,
- Verbindungsmittel (900), die, wenn sie aktiviert sind, den Wagen (1000) mit dem mindestens einen Abdeckelement (12) verbinden, um den Wagen in seine Schaltposition zu verschieben, wenn das mindestens eine Abdeckelement von seiner Öffnungsposition in seine Schließposition zurückgebracht ist, und um den Wagen in seine Position ohne Schalten zu verschieben, wenn das mindestens eine Abdeckelement von seiner Schließposition in seine Öffnungsposition zurückgebracht ist,
- wobei die Verbindungsmittel (900) aktiviert sind, wenn die zweiten Verschiebemittel bei Inhalation durch den Benutzer in die Ausgabeposition verschoben werden, und inaktiviert sind, wenn keine Inhalation erfolgt, so dass eine Öffnung gefolgt von einer Schließung der mindestens einen beweglichen Abdeckung ohne Inhalation vor der Schließung keine Drehung des Schaltrades bewirkt.

2. Vorrichtung nach Anspruch 1, wobei die Verbindungsmittel einen Verbindungsstift (900) aufweisen, der zusammen mit der Spanneinrichtung (800) verschiebbar ist.

3. Vorrichtung nach Anspruch 1 oder 2, wobei das Schaltrad (40) eine Verzahnung (45) aufweist, die mit der Betätigungseinrichtung (1010) des Wagens (1000) zusammenwirkt, wenn sich der Wagen von seiner Schaltposition in seine Position ohne Schalten verschiebt.

4. Vorrichtung nach einem der vorhergehenden Ansprüche, aufweisend zwei Abdeckelemente (11, 12), die miteinander im Eingriff sind.

## Claims

1. A fluid dispenser device, comprising:
• a body (10) provided with a dispenser orifice;
• at least one cover element (11, 12) that is movable between a closed position and an open position;
• a plurality of individual blisters (21) each containing a dose of fluid, such as a pharmaceutical powder, said blisters being formed on an elongate blister strip (20);
• blister opening means for opening a respective full blister each time said opening means are actuated;
• first displacement means for displacing said blister strip so as to bring a full blister to face said opening means before each inhalation, said first displacement means comprising an indexer wheel (40) provided with at least one cavity that receives the blisters;
• second displacement means (90) for displacing said blister strip, said second displacement means pivoting between a non-dispensing position and a dispensing position in which a blister co-operates with said opening means, said indexer wheel (40) being mounted to turn on said second displacement means, such that in the dispensing position, said indexer wheel (40) is displaced against said opening means so as to open the blister arranged in said cavity;
• a cocking member (800) for urging said second displacement means towards said dispensing position, said cocking member being spring-loaded by compressing a spring (50) by opening said at least one cover element;
• blocking means (30) for retaining said second displacement means in the non-dispensing position;
• trigger means (60) for releasing said blocking means and for enabling said second displacement means, and thus said indexer wheel, to be displaced towards said dispensing position, said trigger means being actuated by the user inhaling;
said device being **characterized in that** it comprises:
• a slidable carriage (1000) that is displaceable between a non-indexing position and an indexing position, said non-indexing position corresponding to the open position of said at least one cover element, and the indexing position corresponding to said closed position of said at least one cover element, said carriage including an actuator member (1010) that co-operates with said indexer wheel (40) so at to turn it when said carriage is displaced from its indexing position towards its non-indexing position;
• connection means (900) which, when they are activated, connect said carriage (1000) with said at least one cover element (12), so as to displace said carriage towards its indexing position when said at least one cover element is brought from its open position towards its closed position, and so as to displace said carriage towards its non-indexing position when said at least one cover element is brought from its closed position towards its open position; and
• said connection means (900) being activated while said second displacement means are being displaced towards said dispensing position while the user is inhaling, and being deactivated in the absence of any inhalation, such that opening said at least one movable cover and then closing it without inhaling does not cause said indexer wheel to turn.

2. A device according to claim 1, wherein said connection means comprise a connection finger (900) that is displaceable together with said cocking member (800).

3. A device according to claim 1 or claim 2, wherein said indexer wheel (40) includes a set of teeth (45) that co-operate with the actuator member (1010) of said carriage (1000) when said carriage is displaced from its indexing position towards its non-indexing position.

4. A device according to any preceding claim, including two cover elements (11 12) that are meshed together.
